(19) **Europäisches Patentamt / European Patent Office / Office européen des brevets**

(11) **EP 3 373 805 B1**

(12) **EUROPEAN PATENT SPECIFICATION**

(45) Date of publication and mention
of the grant of the patent:
**07.06.2023 Bulletin 2023/23**

(21) Application number: **17798592.6**

(22) Date of filing: **19.04.2017**

(51) International Patent Classification (IPC):
**A61B 5/021** (2006.01)   **A61B 5/00** (2006.01)
**A61B 5/024** (2006.01)   **A61B 5/11** (2006.01)

(52) Cooperative Patent Classification (CPC):
**A61B 5/02116; A61B 5/02416; A61B 5/1122;
A61B 5/6817; A61B 5/6821; A61B 5/6824;
A61B 5/6828;** A61B 2560/0261; A61B 2562/0219

(86) International application number:
**PCT/CN2017/081068**

(87) International publication number:
**WO 2017/198028 (23.11.2017 Gazette 2017/47)**

(54) **A METHOD FOR OBTAINING THE BLOOD PRESSURE OF A PERSON, AND A DEVICE THEREOF**

VERFAHREN ZUR ERMITTLUNG DES BLUTDRUCKS EINER PERSON UND VORRICHTUNG DAFÜR

PROCÉDÉ D'OBTENTION DE LA TENSION ARTÉRIELLE D'UNE PERSONNE ET DISPOSITIF ASSOCIÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.05.2016 HK 16105622**

(43) Date of publication of application:
**19.09.2018 Bulletin 2018/38**

(73) Proprietor: **Well Being Digital Limited
Hong Kong (CN)**

(72) Inventors:
• **WONG, Ming Yip Wallace
New Territories
Hong Kong (CN)**
• **MA, Chor Tin
N.T.
Hong Kong (CN)**

(74) Representative: **Abraham, Richard
Maguire Boss
24 East Street
St. Ives, Cambridgeshire PE27 5PD (GB)**

(56) References cited:
WO-A1-2014/165908     WO-A1-2016/040253
CN-A- 101 990 415     CN-A- 103 079 463
CN-A- 105 358 049     CN-A- 105 578 951
US-A- 4 699 152       US-A1- 2007 055 163
US-A1- 2011 009 718

• **POON C C Y ET AL: "Using the Changes in Hydrostatic Pressure and Pulse Transit Time to Measure Arterial Blood Pressure", 2007 ANNUAL INTERNATIONAL CONFERENCE OF THE IEEE ENGINEERING IN MEDICINE AND BIOLOGY SOCIETY : [EMBC '07] ; LYON, FRANCE, 22 - 26 AUGUST 2007 ; [IN CONJUNCTION WITH THE BIENNIAL CONFERENCE OF THE SOCIÉTÉ FRANÇAISE DE GÉNIE BIOLOGIQUE ET MÉDICAL (SFGB, 22 August 2007 (2007-08-22), pages 2336-2337, XP031336673, ISBN: 978-1-4244-0787-3**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Field of Invention**

This invention relates to an apparatus for monitoring blood pressure, and more particularly to wearable blood pressure monitors.

**Background of the invention**

Blood pressure is measured as part of routine and basic medical examination. Knowing the blood pressure of a subject aids medical diagnosis, and the medical practitioner may be alerted to invisible ailments if the blood pressure of a subject is off limits.

Blood pressure is fully measured when both systolic blood pressure and diastolic blood pressure are measured. Systolic blood pressure refers to pressure in blood vessels when the heart contracts while diastolic blood pressure refers to pressure in blood vessels when the heart is relaxed.

A device for measuring blood pressure is called a sphygmomanometer. There are manual and digital sphygmomanometers. A manual sphygmomanometer comprises a cuff to be applied around the bicep tightly, positioned at roughly the same level as the heart while the subject is seated. The cuff is tightened to strangle the bicep. The tightness is released slowly while the practitioner listens to the flow of pounding blood in the brachial artery at the elbow using a stethoscope. The cuff pressure beginning at which sound of pounding blood may be heard is recorded as the systolic blood pressure. The manual sphygmomanometer is installed with a mercury column to allow the operator to read the cuff pressure. As the cuff pressure is released further, the cuff pressure beginning at which the sound of pounding blood can no longer be heard is recorded as the diastolic blood pressure.

While this method of taking blood pressure is generally accurate, inaccuracies do arise from operator error, poor use of the equipment, and inadequate equipment maintenance.

The digital sphygmomanometer was developed for portability, and for obviating the need to use mercury and the need for operator training. While lacking the fine accuracy of a mercury column, the digital sphygmomanometer has been found accurate enough for use in a domestic environment by the subject himself. Most digital sphygmomanometers require a pressurised cuff to be tightened around the bicep of the subject, in the same way as a manual sphygmomanometer. Some digital sphygmomanometers require only the cuffing of the wrist or a finger. In any case, the part which is cuffed must be positioned at the same level as the heart. Both the systolic and diastolic pressures are sensed indirectly using piezoelectric, capacitive or electrostatic pressure sensors. The actual reading is calculated by comparing pressure readings against a calibration. Despite the perceived portability, a somewhat elaborate routine is required for the subject to put on the cuff in order to allow the digital sphygmomanometers to operate. Therefore, the subject must find a seat and a table for resting himself against before he may use a digital sphygmomanometer. This is often inconvenient for a subject who needs to have his blood pressure level measured rather urgently in a crowded urban location. In other words, blood pressure monitors which afford a certain level of privacy and discretion would be welcome.

Accordingly, it is desirable to provide a device and method for evaluating general blood pressure while providing an improved degree of usability, speediness of use, portability or privacy for the user.

Patent publication US2007/055163 (A1) discloses a blood pressure cuff with accelerometers that monitors change of pulse wave at multiple heights, using this data to calculate mean arterial pressure and from it both systolic and diastolic BP. It fails to disclose using a detected pulse wave at a second height in order to calculate systolic blood pressure.

**Summary of the invention**

The invention is defined in the appended claims.

In a first aspect, the invention proposes a method for obtaining the blood pressure of a person comprising steps of monitoring pulsation of blood in a body part of the person while moving the body part from a first height to a second height, detecting a first position in which the intensity of the pulsation of blood changes if the body part moves from the first position in one direction, and in which the intensity of blood pulsation remains generally constant if the body part moves from the first position in the reverse direction, and providing the first position as input to a first calculation model suitable for obtaining the person's blood pressure.

Intensity of pulsation of blood in most embodiments refers to the amplitude of pulsation rather than the frequency of pulsation.

The invention provides a possibility to monitor blood pressure without need of a pressure cuff to be applied around the bicep of the subject. As a result, the invention also provides a possibility of having a truly portable and wearable blood pressure monitor.

In some embodiments, the step of detecting the intensity of pulsation of blood in the body part comprises providing a light source configured for illuminating the body part, providing an optical sensor configured to detect light from the light source which has propagated through the body part, and measuring the amplitude of the propagated light. Alternatively, the step of detecting the intensity of pulsation of blood in the body part comprises providing a tonometer on the body part.

Preferably, the body part is a part of a limb of the person and more preferably his wrist. This provides a wrist-worn pressure monitor which can be worn at all times for someone who requires regular monitoring of blood pressure. Alternatively, the body part is an ear canal of the person. The ear canal is not commonly embellished and is likely to be out of the way of other wearable devices competing for a spot on the body.

Preferably, the detection of a first position comprises a step of detecting an angular displacement of the body part. Angular displacement of a body part such as the rotation of an upper limb about the shoulder as the origin is virtually the same, despite measurements taken from varying locations along the upper limb. Therefore, determining the first position in terms of angular displacement is preferably to change in height, as this allows data of different people of different body height to be cross-referenced for study.

In some embodiments, the first position is level to the person's heart or below the person's heart, and the first position is suitable for obtaining the systolic pressure of the person. In other embodiments, the first position is above the person's heart, and the first position is suitable for obtaining the diastolic pressure of the person.

However, it is preferred that the method for obtaining the blood pressure of a person further comprises steps of monitoring pulsation of blood in the body part of the person while moving the body part from a third height to a fourth height, detecting a second position in which the intensity of the pulsation of blood changes if the body part moves from the second position in one direction, and in which the intensity of blood pulsation remains generally constant if the body part moves from the second position in the reverse direction, and providing the second position as input to a second calculation model suitable for obtaining the person's blood pressure, wherein the first position is suitable for obtaining one of either the systolic pressure of the person or the diastolic pressure of the person, and the second position is suitable for obtaining the other one of either the systolic pressure of the person or the diastolic pressure of the person.

Therefore, embodiments which only measures one of either the systolic pressure of the person or the diastolic pressure of the person, and embodiments which measure both the systolic pressure of the person and the diastolic pressure of the person are within the contemplation of the invention.

In a second aspect, the invention proposes a blood pressure monitor suitable for being worn on a body part of a person, comprising a blood pulsation monitor, a movement detector configured to detect a first height position of the body part in which the blood pulsation monitor observes that intensity of pulsation of blood in the body part changes if the body part moves from the first height position in one direction, and in which the blood pulsation monitor observes that pulsation of blood remains generally constant if the body part moves from the first height position in the reverse direction, and a data treatment module for calculating the person's blood pressure based on the first height position.

Intensity of pulsation of blood in most embodiments refers to the amplitude of pulsation rather than the frequency of pulsation.

Optionally, the data treatment module is capable of wirelessly communicating the first height position to a remote computing module for calculating the person's blood pressure based on the first height position.

Optionally, the blood pulsation monitor comprises a light source configured for illuminating the body part, an optical sensor configured to detect light from the light source which has propagated through the body part in order to detect the intensity of pulsation of blood in the body part. Alternatively, the blood pulsation monitor comprises a tonometer to detect the intensity of pulsation of blood in the body part.

Preferably, the body part is an upper limb of the person and more preferably one of his wrists. The wrist is a convenient location for wearing a device comfortably for a long time. This provides that the blood pulsation monitor may be used as a long term wearable device.

In some embodiments, the blood pressure monitor is configured to operate as one of a pair of blood pressure monitors, wherein the first one of the pair is configured to be worn on one location on the body part and the second one of the pair is configured to be worn adjacent the first one of the pair on the body part.

In other embodiments, the body part is an ear canal of the person.

Optionally, the movement detector is also configured to detect a second height position of the body part in which the blood pulsation monitor observes that intensity of pulsation of blood in the body part changes if the body part moves from the second height position in one direction, and in which the blood pulsation monitor observes that pulsation of blood remains generally constant if the body part moves from the second height position in the reverse direction, and the data treatment module also being suitable for calculating the person's blood pressure based on the second height position, in which the first position is based upon for calculating one of either the systolic pressure of the person or the diastolic pressure of the person, and the second position is based upon for calculating the other one of either the systolic pressure of the person or the diastolic pressure of the person.

**Brief description of the Figures**

It will be convenient to further describe the present invention with respect to the accompanying drawings that illustrate possible arrangements of the invention, in which like integers refer to like parts. Other arrangements of the invention are possible, and consequently the particularity of the accompanying drawings is not to be understood as superseding the generality of the preceding description of the invention.

Figure 1 shows a first embodiment of the invention;
Figure 2 shows a signal obtained by the embodiment of Figure 1;

Figure 3 shows a standard cardiogram for explaining the signal of Figure 2;

Figure 4 shows how the embodiment of Figure 1 is used by a user;

Figure 5 shows how signals obtain by the embodiment of Figure 1;

Figure 5a shows how a mathematical model fitting may be used in the embodiment of Figure 5;

Figure 6 shows how signals obtained, as illustrated in Figure 5, are applied against a calibration;

Figure 7 shows an alternative to the embodiment of Figure 4;

Figure 8 is a schematic diagram of the parts within the embodiment of Figure 1 ;

Figure 9 shows a further alternative to the embodiment of Figure 4;

Figure 10 shows a further alternative the calibration in Figure 6;

Figure 11 shows a flowchart of the method employed in the embodiment of Figure 1;

Figure 12 shows another embodiment alternative to that of Figure 1;

Figure 13 shows yet another embodiment alternative to that of Figure 1 ;

Figure 14 shows yet another embodiment alternative to that of Figure 1;

Figure 15 shows yet another embodiment alternative to that of Figure 1;

Figure 16 illustrates a part of the embodiment of Figure 15; and

Figure 17 further illustrates the part shown in Figure 16.

**Detailed description of the invention**

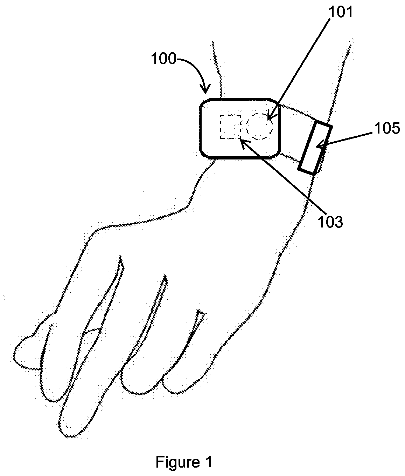

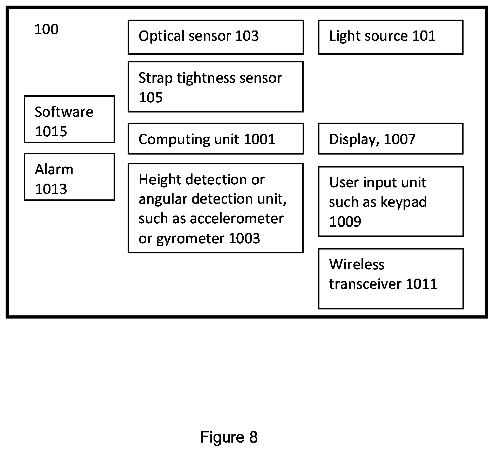

Figure 1 shows a wrist wearable blood pressure monitor 100 shaped like a watch. The underside of the blood pressure monitor 100 comprises a PPG (photoplethysmocharty) sensor. Figure 8 is a schematic diagram showing some functional modules which are preferably provided within the blood pressure monitor 100.

A typical PPG sensor comprises at least one light source 101 such as an LED (light emitting diode), which is illustrated by the circle in broken line, and at least one corresponding optical sensor 103 normally placed next to the light source 101, which is illustrated by the square in broken line. The broken lines represent the invisibility of the light source 101 and the optical sensor 103 from the side of the blood pressure monitor 100 facing away from the wearer.

The blood pressure monitor 100 has a strap by which it may be worn on the wrist. It is advisable that the PPG is tightly secured against the wrist in order to avoid ambient light from affecting detections of the optical sensor 103. The strap is designed to apply a known, predetermined pressure around the wrist. The pressure is predetermined by using a pressure sensor 105 provided on the strap. An example of such a pressure sensor 105 is a MEMS (microelectromechanical systems) barometer. The MEMS barometer operates as a mechanical pressure release device, which releases pressure slowly when the strap is overly tightened around the wrist until the pre-determined pressure remains. Alternatively, the pre-determined pressure is provided by using a pre-selected material for the strap, the material having a specific elasticity or resilience which repeatedly applies the same pressure around the wrist every time the blood pressure monitor 100 is worn. Whichever the methods used, the same pressure is applied to the wrist repeatedly every time the same person wears the blood pressure monitor 100.

Light emitted by the light source 101 into the wrist is scattered in all directions by wrist tissue. A portion of the scattered light propagates towards the optical sensor 103. Blood, skin and tissue all absorb a portion of the light. However, the effect of skin and tissue on light propagation is consistent and the extent of light absorbed by skin and tissue does not change noticeably. The amount of blood in the wrist pulsates as the heart pumps. Hence, the amount of light absorbed when the wrist is full of blood is more than the amount of light absorbed when the tissue is relatively depleted of blood.

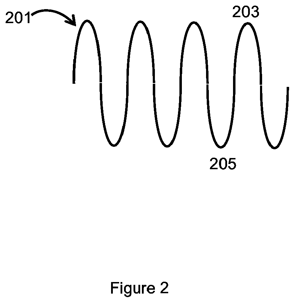

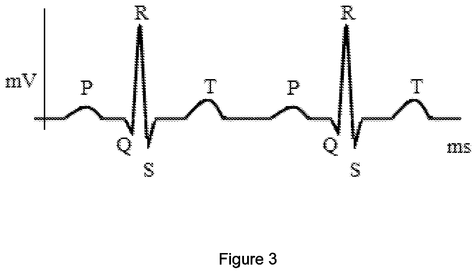

Figure 2 schematically illustrates the pulsating pattern of light propagation through the person's wrist. The pulsation is cyclical and virtually periodic, corresponding to the heart beat. The peaks 203 in the signal 201 are moments when the heart is relaxed and wrist tissue is relatively depleted of blood, such that more light propagates through the wrist to reach the optical sensor 103. Figure 3 shows a standard cardiogram having several peaks which can be detected as electric signals from the different heart chambers, represented by PQRST. Each peak 203 of the signal 201 in Figure 2 corresponds to a period between two adjacent R peaks.

The troughs 205 in the signal 201 in Figure 2 are moments when the heart contracts and pumps blood into the body, and the wrist tissue is full of blood. A lower amount of light reaches the optical sensor 103 because a significant portion of light from the light source 101 has been absorbed by blood.

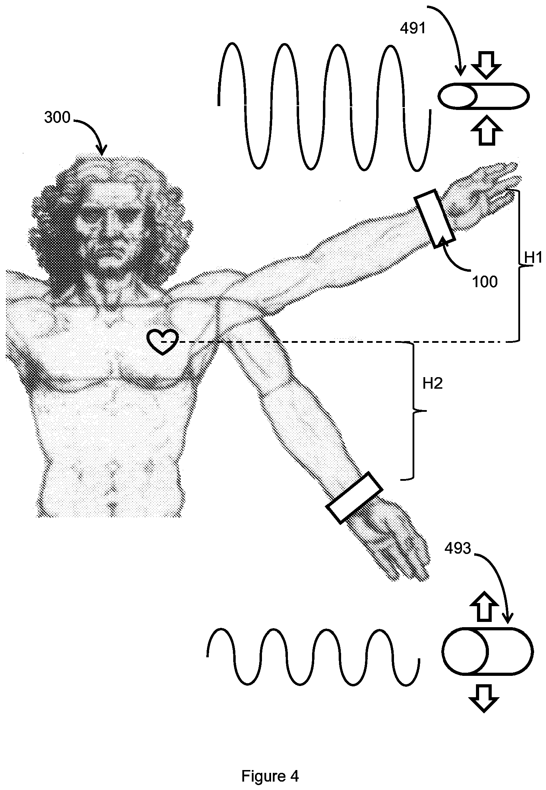

Figure 4 shows a person 300 wearing the blood pressure monitor 100 of Figure 1. The strap of the monitor 100 is tied and tightened around his wrist. The consistent pressure applied by the strap acts as a counter-pressure against pressure in blood vessels within the wrist. The counter-pressure is applied as long as the blood pressure monitor 100 is worn. The counter-pressure causes blood vessels within the wrist to deform slightly. The extent of deformation depends on pressure within the blood vessels and affects the amount of blood which may be pumped into the wrist.

It is well-known that pressure is height-related. There is lower pressure in greater heights above the ground, and there is greater pressure nearer the ground. The same phenomenon can be seen within the wrist. When the person 300 lifts his hand into an elevated position above his heart, blood pressure in his wrist will be lower. A lower blood pressure is relatively weaker against the constant counter-pressure applied by the strap. Therefore, blood vessel deformation due to the counter-pressure from the strap is more pronounced, see label 491 in Figure 4, and a smaller amount of blood is pumped into blood vessels in the wrist.

Conversely, when the person 300 lowers his hand into a position below his heart, the pressure in blood vessels within the wrist is relatively greater. The greater blood pressure exerting against the counter-pressure allows blood vessels within the wrist to recover a little from their deformation, see label 493 in Figure 4, and more blood is pumped into blood vessels in the wrist.

Accordingly, Figure 4 also shows two light propagation signals accompanying the respective blood vessel deformations labelled 491 or 493. The signal at the top of Figure 4 has greater pulsation amplitudes as the wrist has less blood in the elevated hand position; more light is able to propagate through wrist tissue to reach the optical sensor 103. The signal at the bottom of Figure 4 has smaller pulsation amplitudes as the wrist has more blood; more blood in the wrist means more of light is absorbed and less light is able to propagate through wrist tissue to reach the optical sensor 103.

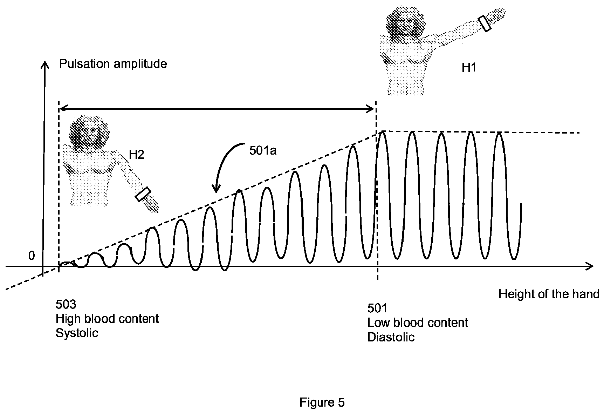

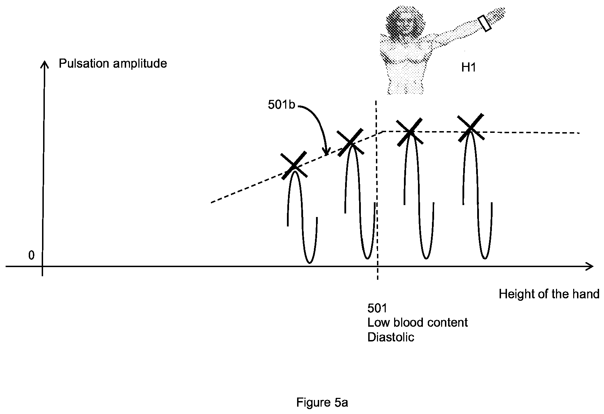

Figure 5 shows how systolic pressure and diastolic pressure are determined by elevated hand position and lower hand position. The left side of the horizontal axis shows the lower hand position and the right side of the horizontal axis shows the elevated hand position.

To begin monitoring his blood pressure, the person 300 gets himself into a ready position by standing upright and stretching out his hand wearing the pressure monitor 100. The hand is stretched away from his side and lifted to shoulder level. Subsequently, when the person moves his hand down from shoulder level and closes it towards his side, and his wrist eventually moves below the heart. Pressure within blood vessels in the hand increases as he moves his hand downwards and blood pumped into the wrist increases steadily, see the gradient labelled 501a. Thus, the pulsation amplitude of light propagating through wrist tissue reduces as the hand is placed lower. When the hand is lowered to a certain point below the heart, pressure within blood vessels in the wrist is able to overcome the counter-pressure and blood pumped in the wrist is at a maximum amount. At this point, the pulsation amplitude of the light propagating through wrist tissue reaches zero, which is where the gradient of the graph 501a crosses the horizontal axis. The height of the wrist at this crossing as indicated by the dashed line labelled 503 is the height of the wrist at which systolic pressure is manifested. In the unlikely event that the hand has moved and closed against the side of the person 300 but the gradient 501a has not crossed the horizontal axis, the gradient can be extrapolated to do so.

Starting again from shoulder level, the person now moves his out-stretched hand upward. As the hand moves upwards, blood pressure decreases and less blood is pumped in the wrist due to the counter-pressure from the strap. See again gradient labelled 501a. Eventually, the blood pressure is so low that only the diastolic pressure remains in the raised hand. Therefore, when the hand is raised to a certain point above the heart, a steady-state appears: blood pumped into the hand is at a minimum even if the hand is raised further. The diastolic blood pressure provides this minimum blood content in the hand, which translates into minimum light absorption and hence maximum light transmission. The point labelled 501 at which the pulsation of light through the wrist begins to show a steady, maximum amplitude represents the height of the wrist at which diastolic pressure is manifested.

The hand does not need to be raised starting with the wrist below heart level. The hand may be raised starting from shoulder level even though the shoulder is already above the heart. This is because the hand normally needs to be raised well above the shoulder to manifest the diastolic pressure.

The skilled man understands that by steady maximum amplitude and steady minimum amplitude, natural variation of signal amplitudes falling above and below the mean levels may be observed.

The actual hand positions for manifesting both diastolic pressure and systolic pressure is actually measured from the wrist wearing the blood pressure monitor 100, and the term 'hand' is used loosely herein.

It is most easily appreciated if the height of the wrist is measured against the ground. Theoretically, however, the height of the wrist may be referenced from a selected point representing the person's heart position instead. Figure 4 illustrates this option, showing an elevated wrist height expressed as H1. According to the invention, H1 is the distance between the height of the wrist and the height of the heart that manifests the diastolic pressure, That is, H1 is measured perpendicularly to the ground. Furthermore, Figure 4 shows a lowered wrist height expressed as H2, where, according to the invention, H2 is the distance between the height of the wrist and the height of the heart that manifests the systolic pressure. The blood pressure monitor 100 described so far may be used to determine diastolic and systolic blood pressures of a person generally, and may be provided to users as such. However, to evaluate blood pressure more accurately, the amplitude of the light propagating through the wrist may be referenced against a mathematical model or a calibration.

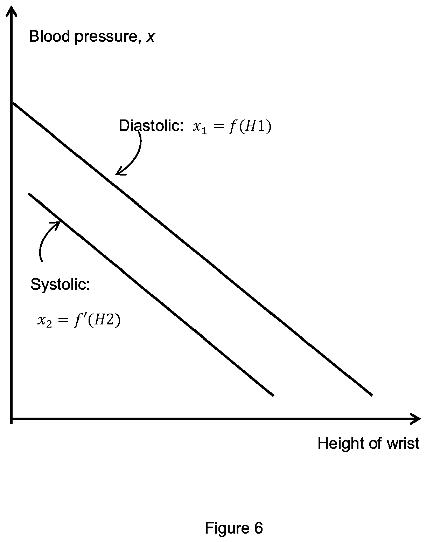

The concept of calibration is illustrated by a schematic mathematical model in Figure 6. The horizontal axis of the graph shown in Figure 6 represents the height of the wrist. The vertical axis represents blood pressure deduced from the height of the wrist. The relationship between the two axes is described by the following equations, (1a) and (1b).

$$x_1 = f(h) \text{ -------------- } (1a)$$

where

x₁ = *diastolic pressure,* defined as the minimum value of blood pressure
h= height of elevation (which is H1 in Figure 4)

$$x_2 = f'(h) \text{ -------------- } (1b)$$

where

x₂ = *systolic pressure*, defined as the maximum value of blood pressure
h = height of elevation (which is H2 in Figure 4)

The two functions, *f* and *f'*, typically give graphs of similar shapes.

As explained using Figure 5, H1 is found by the person elevating his hand to discover the point labelled 501 at which light propagation begins to show steady maximum amplitude. H2 is found by the person lowering his hand to discover the point labelled 503 at which the amplitude of light propagation is reduced to zero or crosses the horizontal axis. Accordingly, any change in H1 and H2 for the same person may be attributed to change in his blood pressure, provided that the counter-pressure applied by the strap has been repeated with sufficient precision and accuracy.

In practice, the calibration or mathematical model can be obtained by proposing a theory or by observing a sampled population of users wearing the blood pressure monitor 100 and having known systolic and diastolic blood pressures. This relates to statistical methods which do not require any exposition in this specification. In some embodiments, the point labelled 501 may be found using a series of discrete positioning of the hand, and applying a mathematical model to the discrete positioning of the hand. As shown in Figure 5a, the amplitude of the propagated light is observed when the hand is lifted to four different levels of height, marked by crosses. None of the four different levels of height coincide with the point labelled 501. Nevertheless, the point labelled 501 is found by applying a known mathematical model 501b to the crosses, instead of relying on the blood pressure monitor 100 to detect it actually.

As shown in Figure 8, and in accordance with the invention, the blood pressure monitor 100 comprises a computing module 1001 and suitable software module 1015 for calculating the person's blood pressure from H1 and H2, and a display screen 1007 for displaying the person's systolic and diastolic blood pressures. Alternatively, the blood pressure monitor 100 relies on a remote device to calculate the systolic and diastolic blood pressures instead of any computing module contained within itself 100. The remote device can be a smart phone which is in wireless communication with the blood pressure monitor 100, and which is able to obtain H1 and H2 from the blood pressure monitor 100 to calculate the person's systolic blood pressure and diastolic blood pressures. In this case, the blood pressure monitor 100 comprises a wireless transceiver 1011 to communicate with a smart phone.

Optionally, an accelerometer or its likes 1003 may be used to determine the exact height that the hand has been raised or lowered to. Of course, an operator may also measures H1 and H2 manually, and enters H1 and H2 into the blood pressure monitor 100 via a keypad 1009 provided on the blood pressure monitor 100.

Figure 6 illustrates equation (1a) and equation (1b) as linear. However, the linearity is simply an illustration. The case may well be that the equations are non-linear. It suffices for the skilled reader to note that actual blood pressure of a person 300 may be expressed as functions of H1 and H2.

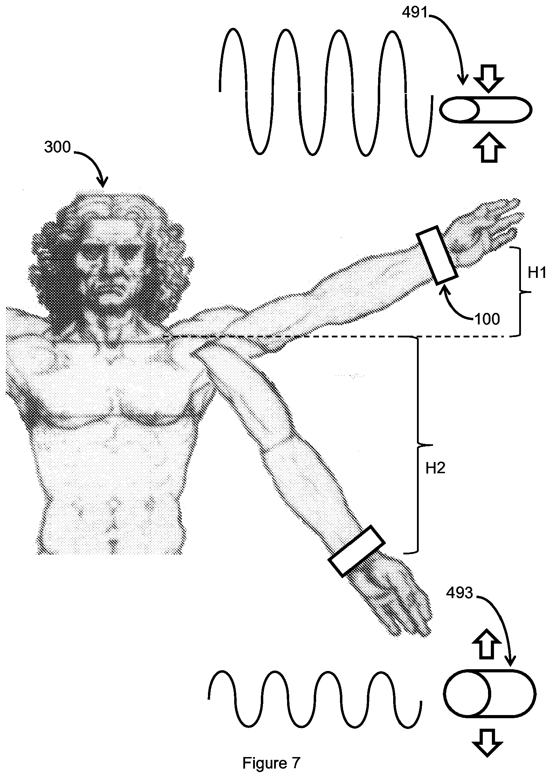

Figure 7 shows a modification of the embodiment of Figure 4, wherein H1 and H2 are measured against the shoulder level of the person 300 instead of the ground or from a point representing his heart. This is a more convenient variation of the embodiment as the person's shoulder is more visible than his heart, and the diastolic pressure and systolic pressure normally manifest in wrist positions well above and below the shoulder. The skilled reader will understand that equation (1a) and equation (1b) may be easily adapted to this varied definition of H1 and H2.

One advantage of the described embodiments is that they provide the possibility of using a PPG sensor to observe pulsation of blood and then to calculate blood pressure from the pulsation. One of the reason why this can be done lies in the use of a PPG sensor to take different readings from the same body part in different positions. Tissue make-up of the person 300 is the same whether a body part is placed in any position. Therefore, the effects of skin and tissue on propagation of light may be eliminated by taking readings in the different positions, and any change in light propagation between the positions is due to blood content in the body part.

An advantage of using a distance between two different wrist positions to determine blood pressure is the possibly greater precision which may be had over a mercury column for reading blood pressure. Blood pressure observed by measurement of body fluid, assumed to be equivalent to water, may be more precise due to greater measurement graduation than that of a mercury column. Mercury has a relative density of 13.56 to water, and therefore, any error of 13.56mm in the wrist position to the shoulder or heart will translate to 1mm of error in the mercury column. If a manual reading of the mercury column is mis-read by 2mm, for example, it is the equivalent of mis-reading the position of the wrist by 27.2mm in the present embodiment, which is an error unlikely to escape notice of any operator of the described embodiments.

To ensure that H1 and H2 are measured properly, the accelerometer 1003 or any height detection unit in the blood pressure monitor 100 is used. An alarm 1013 is provided in the blood pressure monitor 100 to issue a suitable calibration alarm if the accelerometer 1003 shows too much deviation at a known height.

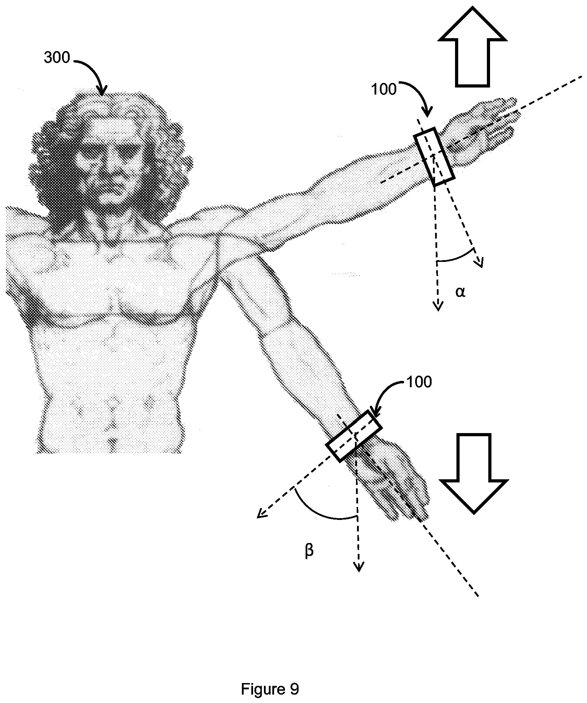

Nevertheless, it may be difficult to measure H1 and H2, whether they be referenced against the ground, the shoulder or the heart. Figure 9 shows a more preferable embodiment which overcomes this difficulty, comprising a gyroscope 1003 installed inside the blood pressure monitor 100 to detect angular deviation from the true perpendicular to the ground. The gyroscope 1003 is tuned to be aligned to the true perpendicular pointing to the ground when the wrist wearing the blood pressure monitor 100 is outstretched horizontally. Therefore, when the person wearing the embodiment of Figure 9 raises his hand from the heart level, the gyroscope is able to detect an angular movement of the blood pressure monitor 100.

When the person stretches his hand outright and raises his hand, at the point where the amplitude of the pulsating light propagating through the wrist reaches a steady maximum, which indicates that blood pulsation in the wrist is at the minimum, a first angle $\alpha$ representing the angular deviation of the blood pressure monitor 100 from the true perpendicular is measured. Accordingly, $\alpha$ is the angle at which diastolic pressure manifests. The skilled reader will understand that $\alpha$ can be used to calculate the extent of upward rotation of the hand or upper limb from the horizontal, about the person's shoulder as the origin.

Conversely, when the person stretches his hand outright and then lowers his hand from his shoulder level, at the point where the amplitude of the pulsating light propagating through the wrist is reduced to zero and crosses the horizontal axis, which indicates that blood pulsation in the wrist is at the maximum, a second angle $\beta$ representing another angular deviation of the blood pressure monitor 100 to the true perpendicular is measured, $\beta$ is the angle at which systolic pressure manifests. The skilled reader will understand that $\beta$ can be used to calculate the extent of downward rotation of the hand or upper limb from the horizontal, about the person's shoulder as the origin.

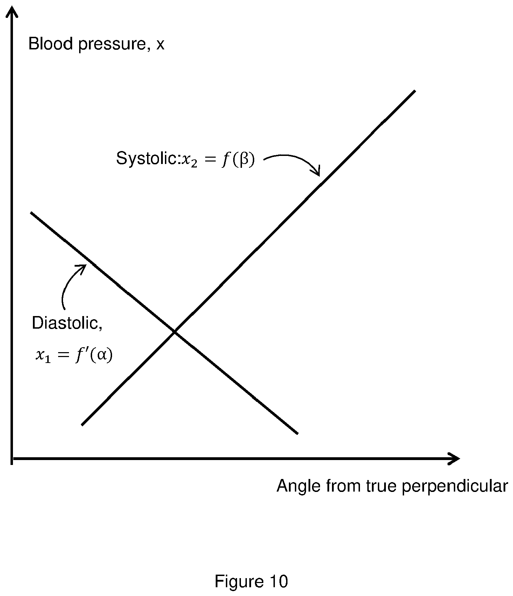

The angles $\alpha$ and $\beta$ are different for people with different blood pressure levels. As illustrated in the graph of Figure 10, a person with high diastolic blood pressure has a small $\alpha$, that is,

$$\alpha \propto \frac{1}{\text{diastolic blood pressure}}$$

The higher the hand needs to be raised above the shoulder to manifest the diastolic pressure, at 501, the greater the angular deviation of $\alpha$ from the true perpendicular, and the lower the person's diastolic blood pressure; the lower need the hand be raised above the shoulder to manifest the diastolic pressure, at 501, the lower the angular deviation of $\alpha$ from the true perpendicular, and the greater the person's diastolic blood pressure.

Conversely, a person with high systolic blood pressure has a greater $\beta$. The lower the hand is dropped below the shoulder (and the heart) to manifest the systolic pressure, at 503, the greater the angular deviation of $\beta$ from the true perpendicular. Accordingly,

$$\beta \propto \text{systolic blood pressure}$$

In other words, the greater $\beta$ the greater the systolic blood pressure; a person with low systolic blood pressure needs only to lower his hand slightly to a relatively small $\beta$ to manifest the systolic blood pressure.

By measuring angular displacement to determine blood pressure, the absolute height of the hand position to the person's heart, shoulder or to the ground does not need to be measured. This is particularly advantageous over the earlier embodiments, as the position on the wrist where the blood pressure monitor 100 is worn may vary easily between different occasions of blood pressure measurement and cause measurements of H1 and H2 to be imprecise. In one application of this embodiment, the person may simply hold on to a door handle and allow himself to stand (so that his hand is below heart level) and squat (so that his hand is above heart level) to determine blood pressure; the gyrometer is able to measure the position of his hand overhead or below the shoulder by angular displacement.

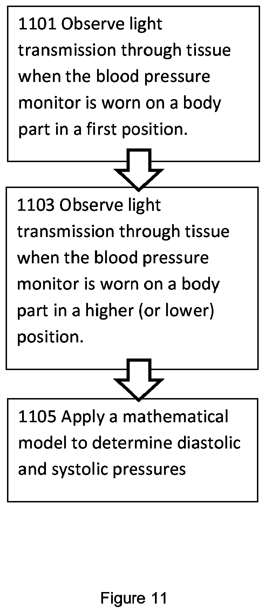

Figure 11 is a flowchart showing one possible general procedure of determining blood pressure using the embodiment of Figure 1. At first, in step 1101 , light propagation level through a body part, which may be the wrist, is read when the body part is in a first position. This may relate to the hand when it is raised in the afore-described embodiments. Then, in step 1103, light propagation level is read again but when the same part is in a second position, and this may relate to the hand when it is lowered. Of course, the reverse in starting from a hand lowered position and then proceeding to a hand raised position is also within the contemplation of the embodiments. The positions of the body part in which the amplitude of light pulsation is at maximum, at 501, and in which amplitude of light pulsation is zero, at 503, are noted and applied against a calibration or mathematical model to determine the diastolic blood pressure and the systolic blood pressure, in step 1105.

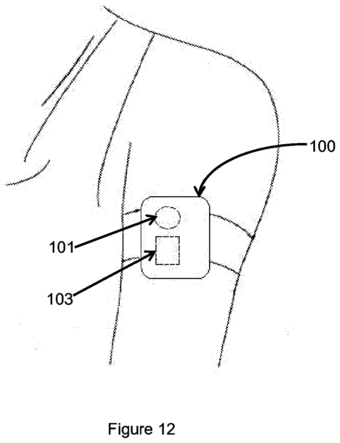

Figure 12 shows another embodiment which is a blood pressure monitor 100 comprising a PPG sensor and is worn around the arm or biceps of the person. In order that this blood pressure monitor 100 may be moved across the heart when the arm is raised and lowered, the blood pressure monitor 100 is preferably worn as near to the elbow as possible. Similar to the afore-described embodiments, H1 and H2 are measured as the distance between the blood pressure monitor 100 and the ground, the person's shoulder or heart level, and a calibration or mathematical model used to calculate blood pressure. Alternatively, the blood pressure monitor 100 may contains a gyrometer for determining angular deviations α and β.

Other parts of limb on the upper body may be used, such as the finger or other parts of the forearm other than the wrist, as long as the part may be lifted above the heart or lowered below the heart.

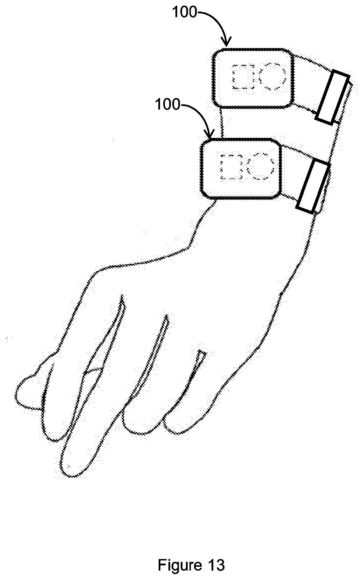

Figure 13 yet shows another embodiment which comprises a pair of blood pressure monitors 100, worn such that a blood pressure monitor 100 is located on the wrist and another one 100 adjacent to the first one 100, on the forearm of the same hand. The configuration of the blood pressure monitor 100 to be worn on the forearm may comprise a longer strap and perhaps a stronger light source 101 for illuminating the thicker tissue layers of the forearm, and perhaps a more sensitive optical sensor 103 for detecting light propagation through the thicker forearm. Any inaccuracies or reading discrepancies between the two blood pressure monitors 100 may be mathematically treated and removed, resulting in a more accurate reading of blood pressure. The benefits of the embodiment of Figure 9 will be more easily appreciated In this embodiment, since the angular displacement from the true perpendicular is the same for both the blood pressure monitors 100 no matter where they are placed along the same limb, whereas H1 and H2 measured from the heart, shoulder or ground are different for the two blood pressure monitors 100 which thus require a different calibration each.

While embodiments have been described as monitoring the amount of light propagating through the person's skin, blood and tissue, the actual measurement may be taken on either light transmission or light absorption.

Where it has been described that measurement of wrist position starts from shoulder level to move below the shoulder level, the skilled reader understands that the direction of movement is a matter of choice, and the wrist may well start moving from the side of the person towards shoulder level. Similarly, other described limb movements may be executed in the direction which is the reverse of that which has been described.

Although drawings provided in this specification shows a sidewise raising and lowering of the person's hand, it is possible that the hand be raised and lowered when stretched to the front of the person.

The amount of light absorbed by blood in the person's tissue depends on the selected frequency of the light used. Therefore, an optimal frequency is typically selected and used in the embodiments for optimal performance. In some embodiments, however, two or more different light frequencies or frequency ranges are used at once to better discriminate blood pulsation reading from the influence of noise contributing factors such as ambient light and so on. For example, one monochromatic near-infrared frequency and a far infrared frequency are used at the same time.

Although embodiments have been described showing a person 300 wearing a blood pressure monitor 100 in an upright position, and moving his hand up and down vertically, it is possible in other embodiments for the person to be wearing the blood pressure monitor 100 lying on a bed. In such embodiments, the person moves his hand wearing the blood pressure monitor 100 from his back to his front, and therefore also moving his hand passed his heart vertically.

In yet a further embodiment, the embodiment is ankle-worn instead of wrist-worn. The person simply lies down on a bed to monitor his blood pressure. One reading is taken when the leg wearing the embodiment is rested on the bed and levelled with the heart, and another when the leg is lifted up into the air and raised above the heart.

Although amplitude of light propagation is used in the afore-mentioned embodiments to monitor pulsation of blood, it is possible that non-optical measurement is used instead. For example, the blood pressure monitor 100 may comprise a tonometer in place of the PPG. A tonometer is an instrument for measuring the pressure in a part of the body or a blood vessel. The amplitude of the pulsation can be correlated to the blood pressure in a similar way as the pulsating amplitude of propagated light. The skilled reader will note that a greater amplitude in the afore-described embodiments using a PPG refers to greater light transmission and hence less blood in the wrist whereas, if a tonometer is used, a greater amplitude represent more blood pumped into the wrist. Both calibration and equations as discussed will have to be adapted accordingly.

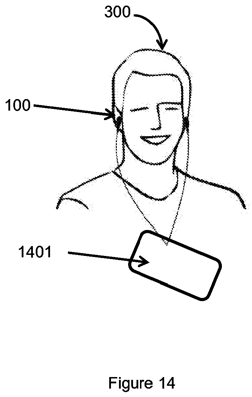

In yet another embodiment, a blood pressure monitor 100 can be provided in the form of an ear worn device, as illustrated in Figure 14. This ear worn device comprises an ear bud which is installed with a light source 101 and an optical sensor 103, as well as a gyrometer. The ear bud is shaped to be suitable for being inserted into the ear canal. The blood pressure monitor 100 has either wired or wireless connection to a device such as a display screen or smart phone 1401 which can display the readings of the blood pressure monitor 100. The display screen or the smart phone 1401 also provides the person 300 with interface for controlling the operation of the blood pressure monitor 100. Furthermore, a smart phone may provide the processing and memory resource for calculating blood pressure based on the readings observed by the optical sensor 103 in the ear bud 1501. This allows the ear bud 1501 to be small, as a processor is not needed to be installed into the ear bud 1501. In contrast, the embodiment of Figure 1 is large enough to contain its own processor and memory, and therefore the embodiments like that which is in Figure 1 may be easily operated with need of an accompanying smart phone. The requirement of a suitable application in the smart phone 1401 for operating the blood pressure monitor 100 is a well-known concept and does not require any exposition in this specification.

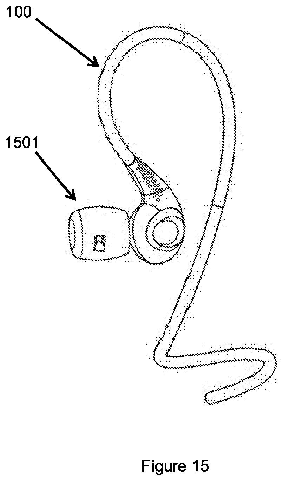

Optionally, it may be the sole function of the blood pressure monitor 100 of Figure 14 to measure the blood pressure of the person 300 wearing it. Alternatively, however, the ear bud type blood pressure monitor 100 is also an ear phone. Figure 15 shows this variation of the embodiment of Figure 14 as a hook-on earphone, the speaker of which is encased in an ear bud 1501 meant for insertion into the ear canal. As with the embodiment of Figure 14, the ear bud 1501 is installed with a light source 101 and sensor for monitoring blood pulsation in the ear canal.

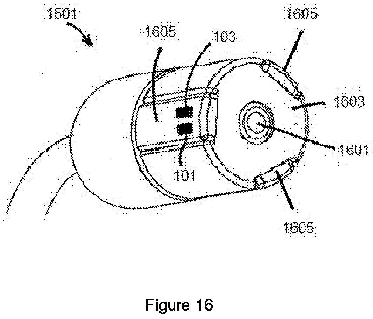

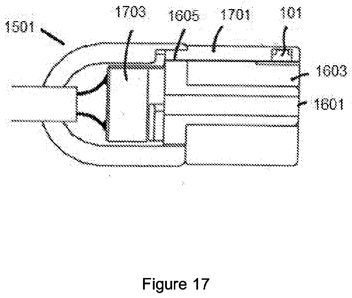

Figure 16 and Figure 17 show how a light source 101 and an optical sensor 103 may be arranged in the ear bud 1501 of the embodiment of Figure 15. Typically, the ear bud 1501 is made of a deformable resilient outer part 1701 sized to fit within the ear canal of a person.

Figure 16 illustrates the core parts of the ear bud 1501 with the resilient outer part removed. Figure 17 shows the resilient outer part 1701 assembled into the embodiment.

Within the ear bud 1501 is a speaker 1703, a hollow inner core 1601 for sound conduction from the speaker in to the ear, an resilient inner foam structure 1603 for softness and flexibility, thin wirings (not illustrated) for connection to the light source 101 and optical sensor 103. The resilient outer part 1701 provides increased comfort and protection of the light source 101 and optical sensor 103s. The resilient inner foam 1603 may be compressed during insertion of the ear bud 1501 into the ear to provide further support in the ear canal. Figure 16 illustrates three sets 1605 of light source 101 and optical sensor 103 pairs, spaced 120 degree around the ear bud. One set is facing the reader showing clearly the light source 101 and optical sensor 103, while the other sets face away from the reader.

To obtain the blood pressure of the person 300 wearing the embodiment 100 of Figure 14 or Figure 15, the person 300 lies down to take a first reading of light propagation through his ear tissue, and then sits up to take another reading of light propagation through his ear tissue. In other words, change in blood pulsation in the ear canal between the positions of lying down and sitting up is noted by observing an accompanying change in the amplitude of light propagation through ear tissue. When the person is lying down, the ear is just about level with the heart and has a similar effect on blood pressure within the ear canal as if the ear canal is below heart level. When the person sits up, the ear is well above the heart. Therefore, this embodiment also comprises a gyroscope to detect the lying down and sitting up of the person 300 and to obtain α and β for calculating the systolic pressure and diastolic blood pressure, or to obtain the height of the ear canal when the person 300 sits up to determine H1 to calculate diastolic pressure. In this embodiment, there is no counter-pressure applied against wall of the ear canal.

It is also possible that a tonometer is used in the ear canal instead of the light source 101 and optical sensor 103.

Although a human has been described as the subject for blood pressure monitoring, the embodiments may be adapted to monitor the blood pressure of any animal which is capable of wearing a device configured to detect blood pulsation, and wherein the body part is capable of moving between two positions relative to the animal's heart.

Accordingly, as one of the simplest embodiment, a method has been described for obtaining the blood pressure of a person 300 comprising steps monitoring pulsation of blood in a body part of the person while moving the body part from a first height to a second height, detecting a first position in which the intensity of the pulsation of blood changes if the body part moves from the first position 501 (or 503) in one direction, and in which the intensity of blood pulsation remains generally constant if the body part moves from the first position 501 (or 503) in the reverse direction, and providing the first position as input to a first calculation model suitable for obtaining the person's blood pressure.

Furthermore, as another one of the simplest embodiment, a blood pressure monitor 100 suitable for being worn on a body part of a person 300 has been described, which comprises a blood pulsation monitor, a movement detector configured to detect a first height position of the body part in which the blood pulsation monitor observes that intensity of pulsation of blood in the body part changes if the body part moves from the first height position 501 (or 503) in one direction, and in which the blood pulsation monitor observes that pulsation of blood remains generally constant if the body part moves from the first height position 501 (or 503) in the reverse direction, and a data treatment module for calculating the person's blood pressure based on the first height position.

While there has been described in the foregoing description preferred embodiments of the present invention, it will be understood by those skilled in the technology concerned that many variations or modifications in details of design, construction or operation may be made without departing from the scope of the present disclosure . The scope of protection is defined solely by the appended claims.

## Claims

1. A method for obtaining the blood pressure of a person comprising steps of:
   with a blood pulsation monitor (101, 103), monitoring pulsation of blood in a body part of the person while moving the body part from a first height to a second height; **characterised by**:

      with a movement detector (1003), detecting a first height position above the person's heart in which: a) the amplitude of the pulsation of blood changes if the body part moves from the first height position in one direction; and b) the amplitude of blood pulsation remains generally constant if the body part moves from the first height position in the reverse direction; and with a data treatment module (1015), providing the first height position as an input to a first calculation model suitable for obtaining the diastolic pressure of the person; monitoring pulsation of blood in the body part while moving the body part from a third height to a fourth height; with the movement detector (1003), detecting a second height position below the person's heart in which a) the amplitude of the pulsation of blood changes if the body part moves from the second height position in one direction; and b) the amplitude of blood pulsation remains generally constant if the body part moves from the second height position in the reverse direction; and

with the data treatment module (1015), providing the second height position as an input to a second calculation model suitable for obtaining the systolic pressure of the person.

2. A method for obtaining the blood pressure of a person as claimed in claim 1, wherein the step of detecting the amplitude of pulsation of blood in the body part comprises:

   providing a light source (101) configured for illuminating the body part;
   providing an optical sensor (103) configured to detect light from the light source (101) which has propagated through the body part; and
   measuring the amplitude of the propagated light.

3. A method for obtaining the blood pressure of a person as claimed in claim 1, wherein
   the step of detecting the amplitude of pulsation of blood in the body part comprises:
   providing a tonometer on the body part.

4. A method for obtaining the blood pressure of a person as claimed in any one of claim 1 to claim 3, wherein the body part is a part of a limb of the person.

5. A method for obtaining the blood pressure of a person as claimed in 4, wherein the part of a limb is a wrist of the person.

6. A method for obtaining the blood pressure of a person as claimed in any one of claim 1 to claim 3, wherein the body part is an ear canal of the person.

7. A method for obtaining the blood pressure of a person as claimed in any one of the preceding claims, wherein
the detecting of a first height position comprises a step of:
detecting an angular displacement of the body part.

8. A blood pressure monitor (100) suitable for being worn on a body part of a person, comprising:

   a blood pulsation monitor (101, 103);
   a movement detector (1003) configured to detect a height position of the body part;
   **characterised in that** the movement detector (1003) is configured to:

      detect a first height position of the body part above the person's heart in which the blood pulsation monitor (101, 103) observes that: a) amplitude of pulsation of blood in the body part changes if the body part moves from the first height position in one direction; and b) amplitude of pulsation of blood remains generally constant if the body part moves from the first height position in the reverse direction; and
      detect a second height position of the body part below the person's heart in which the blood pulsation monitor (101, 103) observes that: a) amplitude of pulsation of blood in the body part changes if the body part moves from the second height position in one direction; and b) amplitude of pulsation of blood remains generally constant if the body part moves from the second height position in the reverse direction; and
      a data treatment module (1015) operative to calculate the diastolic pressure of the person based on the first height position and operative to calculate the systolic pressure of the person based on the second height position.

9. A blood pressure monitor (100) suitable for being worn on a body part of a person as claimed in claim 8, wherein
   the data treatment module (1015) is capable of wirelessly communicating the first height position to a remote computing module for calculating the person's blood pressure based on the first height position.

10. A blood pressure monitor (100) suitable for being worn on a body part of a person as claimed in any one of claim 8 to claim 9, wherein
    the blood pulsation monitor (101, 103) comprises:

    a light source (101) configured for illuminating into the body part;

an optical sensor (103) configured to detect light from the light source (101) which has propagated through the body part.

11. A blood pressure monitor (100) suitable for being worn on a body part of a person as claimed in any one of claim 8 to claim 10, wherein

the blood pulsation monitor comprises a tonometer.

12. A blood pressure monitor (100) suitable for being worn on a body part of a person as claimed in any one of claim 8 to claim 11, wherein the body part is the person's wrist.

13. A blood pressure monitor (100) suitable for being worn on a body part of a person configured to operate as one of a pair of blood pressure monitors each as claimed in any one of claim 8 to claim 12; wherein the first one of the pair is configured to be worn on one location on the body part and the second one of the pair is configured to be worn adjacent the first one of the pair on the body part.

14. A blood pressure monitor (100) suitable for being worn on a body part of a person as claimed in any one of claim 8 to claim 11, wherein the body part is an ear canal of the person.

15. A blood pressure monitor (100) suitable for being worn on a body part of a person as claimed in any one of claim 8 to claim 14, wherein

the movement detector (1003) comprises a gravity sensor capable of detecting angular displacement of the body part; and the first height position is represented by angular displacement of the body part.

**Patentansprüche**

1. Verfahren zur Ermittlung des Blutdrucks einer Person, umfassend die folgenden Schritte:

mit einem Blutpulsationsmonitor (101, 103), Überwachen der Pulsation des Blutes in einem Körperteil der Person, während der Körperteil von einer ersten Höhe zu einer zweiten Höhe bewegt wird;
**gekennzeichnet durch**:

mit einem Bewegungsdetektor (1003), Detektieren einer ersten Höhenposition über dem Herzen der Person, in der: a) sich die Amplitude der Pulsation des Blutes ändert, wenn sich der Körperteil von der ersten Höhenposition in einer Richtung bewegt; und b) die Amplitude der Blutpulsation im Allgemeinen konstant bleibt, wenn sich der Körperteil von der ersten Höhenposition in der umgekehrten Richtung bewegt; und mit einem Datenbehandlungsmodul (1015), Bereitstellen der ersten Höhenposition als Eingabe für einen ersten Berechnungsmodus, der geeignet ist, um den diastolischen Druck der Person zu ermitteln; Überwachen der Pulsation des Blutes in dem Körperteil, während der Körperteil von einer dritten Höhe zu einer vierten Höhe bewegt wird; mit dem Bewegungsdetektor (1003), Detektieren einer zweiten Höhenposition unterhalb des Herzens der Person, in der a) sich die Amplitude der Pulsation des Blutes ändert, wenn sich der Körperteil von der zweiten Höhenposition in einer Richtung bewegt; und b) die Amplitude der Blutpulsation im Allgemeinen konstant bleibt, wenn sich der Körperteil von der zweiten Höhenposition in der umgekehrten Richtung bewegt; und mit dem Datenbehandlungsmodul (1015), Bereitstellen der zweiten Höhenposition als Eingabe für ein zweites Berechnungsmodell, das geeignet ist, um den systolischen Druck der Person zu ermitteln.

2. Verfahren zur Ermittlung des Blutdrucks einer Person nach Anspruch 1, wobei der Schritt des Detektierens der Amplitude der Pulsation des Blutes in dem Körperteil Folgendes umfasst:

Bereitstellen einer Lichtquelle (101), die konfiguriert ist, um den Körperteil zu beleuchten; Bereitstellen eines optischen Sensors (103), der konfiguriert ist, um Licht von der Lichtquelle (101) zu detektieren, das sich durch den Körperteil ausgebreitet hat; und Messen der Amplitude des ausgebreiteten Lichts.

3. Verfahren zur Ermittlung des Blutdrucks einer Person nach Anspruch 1, wobei der Schritt des Detektierens der Amplitude der Pulsation des Blutes in dem Körperteil Folgendes umfasst: Bereitstellen eines Tonometers an dem Körperteil.

4. Verfahren zur Ermittlung des Blutdrucks einer Person nach einem der Ansprüche 1 bis 3, wobei der Körperteil ein Teil eines Gliedes der Person ist.

5. Verfahren zur Ermittlung des Blutdrucks einer Person nach Anspruch 4, wobei der Teil eines Gliedes ein Handgelenk der Person ist.

**6.** Verfahren zur Ermittlung des Blutdrucks einer Person nach einem der Ansprüche 1 bis 3, wobei der Körperteil ein Gehörgang der Person ist.

**7.** Verfahren zur Ermittlung des Blutdrucks einer Person nach einem der vorhergehenden Ansprüche, wobei
das Detektieren einer ersten Höhenposition einen folgenden Schritt umfasst:
Detektieren einer Winkelverschiebung des Körperteils.

**8.** Blutdruckmonitor (100), der geeignet ist, um an einem Körperteil einer Person getragen zu werden, umfassend:

einen Blutpulsationsmonitor (101, 103);
einen Bewegungsdetektor (1003), der konfiguriert ist, um eine Höhenposition des Körperteils zu detektieren;
**dadurch gekennzeichnet, dass** der Bewegungsdetektor (1003) für Folgendes konfiguriert ist:

Detektieren einer ersten Höhenposition des Körperteils über dem Herzen der Person, in welcher der Blutpulsationsmonitor (101, 103) beobachtet, dass: a) sich Amplitude der Pulsation des Blutes in dem Körperteil ändert, wenn sich der Körperteil von der ersten Höhenposition in einer Richtung bewegt; und b) Amplitude der Pulsation des Blutes im Allgemeinen konstant bleibt, wenn sich der Körperteil von der ersten Höhenposition in der umgekehrten Richtung bewegt; und
Detektieren einer zweiten Höhenposition des Körperteils unterhalb des Herzens der Person, in welcher der Blutpulsationsmonitor (101, 103) beobachtet, dass: a) sich Amplitude der Pulsation des Blutes in dem Körperteil ändert, wenn sich der Körperteil von der zweiten Höhenposition in einer Richtung bewegt; und b) Amplitude der Pulsation des Blutes im Allgemeinen konstant bleibt, wenn sich der Körperteil von der zweiten Höhenposition in der umgekehrten Richtung bewegt; und
ein Datenbehandlungsmodul (1015), das bedienbar ist, um den diastolischen Druck der Person basierend auf der ersten Höhenposition zu berechnen, und bedienbar ist, um den systolischen Druck der Person basierend auf der zweiten Höhenposition zu berechnen.

**9.** Blutdruckmonitor (100), der geeignet ist, um an einem Körperteil einer Person getragen zu werden, nach Anspruch 8, wobei
das Datenbehandlungsmodul (1015) in der Lage ist, die erste Höhenposition drahtlos an ein entferntes Rechenmodul zu kommunizieren, um den Blutdruck der Person basierend auf der ersten Höhenposition zu berechnen.

**10.** Blutdruckmonitor (100), der geeignet ist, um an einem Körperteil einer Person getragen zu werden, nach einem von Anspruch 8 bis 9, wobei
der Blutpulsationsmonitor (101, 103) Folgendes umfasst:

eine Lichtquelle (101), die konfiguriert ist, um den Körperteil zu beleuchten;
einen optischen Sensor (103), der konfiguriert ist, um Licht von der Lichtquelle (101) zu detektieren, das sich durch den Körperteil ausgebreitet hat.

**11.** Blutdruckmonitor (100), der geeignet ist, um an einem Körperteil einer Person getragen zu werden, nach einem von Anspruch 8 bis 10, wobei
der Blutpulsationsmonitor ein Tonometer umfasst.

**12.** Blutdruckmonitor (100), der geeignet ist, um an einem Körperteil einer Person getragen zu werden, nach einem von Anspruch 8 bis 11, wobei
der Körperteil das Handgelenk der Person ist.

**13.** Blutdruckmonitor (100), der geeignet ist, um an einem Körperteil einer Person getragen zu werden, der konfiguriert ist, um als einer von einem Paar von Blutdruckmonitoren zu arbeiten, jeder nach einem von Anspruch 8 bis 12; wobei
der erste von dem Paar konfiguriert ist, um an einer Stelle an dem Körperteil getragen zu werden, und der zweite von dem Paar konfiguriert ist, um benachbart zu dem ersten von dem Paar an dem Körperteil getragen zu werden.

**14.** Blutdruckmonitor (100), der geeignet ist, um an einem Körperteil einer Person getragen zu werden, nach einem von Anspruch 8 bis 11, wobei
der Körperteil ein Gehörgang der Person ist.

**15.** Blutdruckmonitor (100), der geeignet ist, um an einem Körperteil einer Person getragen zu werden, nach einem von Anspruch 8 bis 14, wobei

der Bewegungsdetektor (1003) einen Schwerkraftsensor umfasst, der in der Lage ist, Winkelverschiebung des Körperteils zu detektieren; und
die erste Höhenposition durch Winkelverschiebung des Körperteils dargestellt ist.

## Revendications

1. Procédé permettant d'obtenir la tension artérielle d'une personne comprenant les étapes de :

   avec un moniteur de pulsations sanguines (101, 103), surveillance des pulsations du sang dans une partie corporelle de la personne pendant un mouvement de la partie corporelle d'une première hauteur à une seconde hauteur ;
   **caractérisé par** :

   avec un détecteur de mouvement (1003), la détection d'une première position de hauteur au-dessus du coeur de la personne dans laquelle :

   a) l'amplitude de la pulsation du sang change si la partie corporelle bouge de la première position de hauteur suivant une direction ; et
   b) l'amplitude des pulsations sanguines reste généralement constante si la partie corporelle bouge de la première position de hauteur en sens inverse ; et

   avec un module de traitement de données (1015), la fourniture de la première position de hauteur en tant qu'entrée à un premier mode de calcul approprié pour obtenir la pression diastolique de la personne ;
   la surveillance des pulsations du sang dans la partie corporelle tout en bougeant la partie corporelle d'une troisième hauteur à une quatrième hauteur ;
   avec le détecteur de mouvement (1003), la détection d'une seconde position de hauteur en-dessous du coeur de la personne dans laquelle

   a) l'amplitude des pulsations du sang change si la partie corporelle bouge de la seconde position de hauteur suivant une direction ; et
   b) l'amplitude des pulsations sanguines reste généralement constante si la partie corporelle bouge de la seconde position de hauteur en sens inverse ; et

   avec le module de traitement de données (1015), la fourniture de la seconde position de hauteur en tant qu'entrée pour un second modèle de calcul approprié pour obtenir la pression systolique de la personne.

2. Procédé permettant d'obtenir la tension artérielle d'une personne selon la revendication 1, ladite étape de détection de l'amplitude des pulsations du sang dans la partie corporelle comprenant :

   la fourniture d'une source lumineuse (101) conçue pour éclairer la partie corporelle ;
   la fourniture d'un capteur optique (103) conçu pour détecter la lumière provenant de la source lumineuse (101) qui s'est propagée à travers la partie corporelle ; et
   la mesure de l'amplitude de la lumière propagée.

3. Procédé permettant d'obtenir la tension artérielle d'une personne selon la revendication 1, ladite étape de détection de l'amplitude des pulsations du sang dans la partie corporelle comprenant :
   la fourniture d'un tonomètre sur la partie corporelle.

4. Procédé permettant d'obtenir la tension artérielle d'une personne selon l'une quelconque des revendications 1 à 3,
   ladite partie corporelle étant une partie d'un membre de la personne.

5. Procédé permettant d'obtenir la tension artérielle d'une personne selon la revendication 4, ladite partie d'un membre étant un poignet de la personne.

6. Procédé permettant d'obtenir la tension artérielle d'une personne selon l'une quelconque des revendications 1 à 3,
   ladite partie corporelle étant un conduit auditif de la personne.

7. Procédé permettant d'obtenir la tension artérielle d'une personne selon l'une quelconque des revendications précédentes,
   ladite détection d'une première position de hauteur comprenant une étape de :
   détection d'un déplacement angulaire de la partie corporelle.

8. Moniteur de tension artérielle (100) approprié pour être porté sur une partie corporelle d'une personne, comprenant :

   un moniteur de pulsations sanguines (101, 103) ;
   un détecteur de mouvement (1003) conçu pour détecter une position de hauteur de la partie corporelle ;
   **caractérisé en ce que** le détecteur de mouvement (1003) est conçu pour :

   détecter une première position de hauteur de la partie corporelle au-dessus du coeur de la personne dans laquelle le moniteur de pulsations sanguines (101, 103) observe que : a) l'amplitude des pulsations du sang dans la partie corporelle change si la partie corporelle bouge de la première position de hauteur suivant une direction ; et b) l'amplitude des pulsations du sang reste généralement constante si la partie corporelle bouge de la première position de hauteur en sens inverse ; et

détecter une seconde position de hauteur de la partie corporelle en-dessous du coeur de la personne dans laquelle le moniteur de pulsations sanguines (101, 103) observe que : a) l'amplitude des pulsations du sang dans la partie corporelle change si la partie corporelle bouge de la seconde position de hauteur suivant une direction ; et b) l'amplitude des pulsations du sang reste généralement constante si la partie corporelle bouge de la seconde position de hauteur en sens inverse ; et

un module de traitement de données (1015) servant à calculer la pression diastolique de la personne sur la base de la première position de hauteur et servant à calculer la pression systolique de la personne sur la base de la seconde position de hauteur.

9. Moniteur de tension artérielle (100) approprié pour être porté sur une partie corporelle d'une personne selon la revendication 8,
ledit module de traitement de données (1015) pouvant communiquer sans fil la première position de hauteur à un module de calcul distant pour calculer la tension artérielle de la personne sur la base de la première position de hauteur.

10. Moniteur de tension artérielle (100) approprié pour être porté sur une partie corporelle d'une personne selon l'une quelconque des revendications 8 à 9,
ledit moniteur de pulsations sanguines (101, 103) comprenant :
une source lumineuse (101) conçue pour éclairer dans la partie corporelle ; un capteur optique (103) conçu pour détecter la lumière provenant de la source lumineuse (101) qui s'est propagée à travers la partie corporelle.

11. Moniteur de tension artérielle (100) approprié pour être porté sur une partie corporelle d'une personne selon l'une quelconque des revendications 8 à 10,
ledit moniteur de pulsations sanguines comprenant un tonomètre.

12. Moniteur de tension artérielle (100) approprié pour être porté sur une partie corporelle d'une personne selon l'une quelconque des revendications 8 à 11,
ladite partie corporelle étant le poignet de la personne.

13. Moniteur de tension artérielle (100) approprié pour être porté sur une partie corporelle d'une personne conçu pour fonctionner comme un moniteur d'une paire de moniteurs de tension artérielle chacun selon l'une quelconque des revendications 8 à 12 ;
le premier moniteur de la paire étant conçu pour être porté au niveau d'un emplacement sur la partie corporelle et le second moniteur de la paire étant conçu pour être porté à côté du premier moniteur de la paire sur la partie corporelle.

14. Moniteur de tension artérielle (100) approprié pour être porté sur une partie corporelle d'une personne selon l'une quelconque des revendications 8 à 11, ladite partie corporelle étant un conduit auditif de la personne.

15. Moniteur de tension artérielle (100) approprié pour être porté sur une partie corporelle d'une personne selon l'une quelconque des revendications 8 à 14,

ledit détecteur de mouvement (1003) comprenant un capteur de gravité pouvant détecter un déplacement angulaire de la partie corporelle ; et
ladite première position de hauteur étant représentée par un déplacement angulaire de la partie corporelle.

Figure 1

Figure 2

Figure 3

Figure 4

Figure 5

Figure 5a

501
Low blood content
Diastolic

Figure 6

Figure 7

100

| Optical sensor 103 | Light source 101 |
| --- | --- |
| Strap tightness sensor 105 | |

Software 1015

| Computing unit 1001 | Display, 1007 |
| --- | --- |

Alarm 1013

| Height detection or angular detection unit, such as accelerometer or gyrometer 1003 | User input unit such as keypad 1009 |
| --- | --- |
| | Wireless transceiver 1011 |

Figure 8

Figure 9

Blood pressure, x

Systolic:$x_2 = f(\beta)$

Diastolic,
$x_1 = f'(\alpha)$

Angle from true perpendicular

Figure 10

1101 Observe light transmission through tissue when the blood pressure monitor is worn on a body part in a first position.

1103 Observe light transmission through tissue when the blood pressure monitor is worn on a body part in a higher (or lower) position.

1105 Apply a mathematical model to determine diastolic and systolic pressures

Figure 11

Figure 12

Figure 13

Figure 14

100

1501

Figure 15

Figure 16

Figure 17

**EP 3 373 805 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007055163 A1